# EUROPEAN PATENT SPECIFICATION

(11) **EP 1 321 140 B1**
(45) Date of publication and mention of the grant of the patent: **06.04.2005**
(21) Application number: 01958434.1
(22) Date of filing: 23.08.2001
(51) Int. Cl.: A61K 31/192, A61K 9/08, A61K 9/48, A61K 47/34, A61K 47/10, A61K 47/06, A61K 47/08

(54) **IBUPROFEN SOLUTIONS FOR CAPSULE-FILLING AND CAPSULE PREPARATIONS**
IBUPROFEN-LöSUNGEN ALS KAPSELFüLLUNG UND KAPSEL-ZUBEREITUNGEN
SOLUTIONS D'IBUPROFENE DESTINEES A ETRE MISES EN GELULES ET PREPARATIONS EN GELULES

(30) Priority: 25.08.2000 JP 2000255827
(43) Date of publication of application: 25.06.2003
(73) Proprietor: Kowa Company, Ltd., Nagoya-shi, Aichi 460-0003 (JP)
(72) Inventor: KATO, Soichiro, Numazu-shi, Shizuoka 410-0872 (JP); TSUMORI, Katsuyuki, Fujinomiya-shi, Shizuoka 418-0034 (JP); SHINODA, Yasuo, Shizuoka-shi, Shizuoka 420-0867 (JP); INAGI, Toshio, Mishima-shi, Shizuoka 411-0038 (JP)
(74) Representative: Bannerman, David Gardner
(86) International application number: PCT/JP2001/007223
(87) International publication number: WO 2002/015900

(56) References cited:
- EP-A- 0 300 277
- EP-A- 0 672 422
- WO-A-88/02625
- WO-A-94/25009
- WO-A-98/02182
- WO-A1-88/02625
- WO-A1-94/25009
- WO-A1-98/02182
- JP-A- 8 333 246

## Description

### Field of the Invention

The present invention relates to a fill liquid composition for capsule into which ibuprofen is dissolved, and to a capsule preparation without precipitating crystals of ibuprofen when water is mixed in the composition.

### Description of the Related Art

Ibuprofen is a phenylpropionic acid based antipyretic antiphlogistic analgesic and is used as a nonproprietary drug for antipyretic analgesic or a cold remedy in addition to as ethical drugs for chronic articular rheumatism, arthralgia and neuralgia.
Ibuprofen preparations exist in the form of a dragee, a filmed tablet, a granule, or a hard capsule (filled with granules). However, these face a difficulty that the prevention of bitterness of ibuprofen and the quick-activeness of ibuprofen are inconsistent when it is dosed. As a preparation in which the prevention of bitterness of ibuprofen and the quick-activeness of ibuprofen are consistently achieved when it is dosed are realized, a preparation may be formulated that comprises a capsule in which a solution of ibuprofen is filled. As such a capsule preparation, the one that contains ibuprofen alone has already been put into market, which uses a surfactant.

As technologies concerned with dissolution of ibuprofen, there have been known those technologies disclosed in Japanese Patent Application Laid-open Nos. Hei 8-333246, Hei 8-333265, Hei 6-9381, Hei 5-310566 and Hei 9-157162, and Japanese Patent Publication No. Hei 7-116021. Japanese Patent Application Laid-open Nos. Hei 8-333246 and Hei 8-333265 discloses an ibuprofen suspension preparation having mixed therein ibuprofen, menthol and water, and an ibuprofen suspension prepararion in which ibuprofen is mixed with cassia bark extract, ginger extract and water, for the purpose of masking the bitterness. When these suspensions are used as a fill liquid composition for capsules, they would cause softening of the capsule since its water content is 60% or more. In the ibuprofen preparations disclosed in Japanese Patent Application Laid-open Nos. Hei 8-333246 and Hei 6-9381 ibuprofen is not dissolved but exists as a suspension. The ibuprofen preparations disclosed in Japanese Patent Application Laid-open Nos. Hei 6-9381, Hei 5-310566 and Hei 9-157162 contain a surfactant as an indispensable component, so that the softening of the capsule is feared because of the interaction of the surfactant with the capsule film. The ibuprofen preparation disclosed in Japanese Patent Publication No. Hei 7-116021 has a problem in stability of ibuprofen.

As described above, there has been obtained no satisfactory fill liquid compositions for capsules that does not precipitate crystals of ibuprofen when water is mixed therein.

### Summary of the Invention

A preparation in which a solution of ibuprofen has been filled in a capsule has been desired as the preparation in which the prevention of bitterness and the quick-activeness of ibuprofen are consistent when it is dosed. Since capsule preparations use capsules made of gelatin, softening of the capsule is observed unless water is mixed in an amount of less than 20% by weight based on the total weight of the fill liquid composition in the capsule. On the other hand, in hard capsules, cracking of the capsule is observed with lapse of time if water is not mixed in the fill liquid composition in the capsule. In the case of soft capsules, the capsule is hardened depending on the components thereof. Therefore, it is necessary to mix water. However, mixing water in the fill liquid composition having dissolved therein ibuprofen forms precipitation of crystals of ibuprofen. As a result, it has been difficult to prepare a fill liquid composition having dissolved therein ibuprofen that causes no precipitation of crystals of ibuprofen when water is mixed therein.

The present invention has been made in view of the above. An object of the present invention is to provide a fill liquid composition for capsules and a capsule preparation having dissolved therein ibuprofen that do not form precipitation of crystals of ibuprofen when water is mixed therein, in order to exhibit the effects that the prevention of bitterness of ibuprofen and the quick-activeness of ibuprofen are consistently achieved.

The inventors of the present invention have made extensive studies taking the above points into consideration. As a result, they have found out a formulation for a fill liquid composition for capsules having dissolved therein ibuprofen that does not form precipitation of crystals of ibuprofen when water is mixed therein. That is, they have found out that mixing terpenoid in a solution of ibuprofen, polyethylene glycol and water gives rise to a fill liquid composition that does not form precipitation of crystals of ibuprofen when water is mixed therein. The present invention is based on this discovery.

That is, the present invention provides the followings.
(1) A fill liquid composition for capsules comprising ibuprofen, polyethylene glycol, water and terpenoid.
(2) The fill liquid composition described in the above item (1), wherein the content of terpenoid is 0.1 to 15% by weight based on total weight of the fill liquid composition.
(3) The fill liquid composition described in the above item (1) or (2), wherein terpenoid is selected from the group consisting of menthol, limonene, borneol, *dl*-camphor, and peppermint oil.
(4) The fill liqid composition described in the above item (3), wherein terpenoid is menthol or peppermint oil.
(5) The fill liquid composition described in any of the above items (1) to (4), further comprising one or more drugs selected from antipyretic analgesic, anti-histamine, antitussive, expectorant, sympathetic nerve stimulant, analeptic, hypnotic sedative, and antiphlogistic.
(6) An ibuprofen preparation comprising a capsule and the fill liquid composition described in any of the above items (1) to (5) filled in the capsule.

### Detailed Description of the Invention

Hereinafter, the present invention is illustrated in detail below.

The fill liquid composition of the present invention is a mixture comprising ibuprofen, polyethylene glycol, water and terpenoid.

Usually, the daily dosage of ibuprofen is 200 to 600 mg, preferably 300 to 600 mg, or more preferably 400 to 600 mg. The amount of ibuprofen mixed in the fill liquid composition may be adjusted appropriately so that the dosage is in the above-mentioned range depending on the amount of the fill liquid composition per capsule (usually 1 mL or less, preferably 0.7 mL or less, per capsule) and daily dose number (for example, 3 to 6 capsules/day). Specifically, the mixing amount of ibuprofen is usually 5 to 50% by weight, preferably 10 to 25% by weight, or more preferably 15 to 20% by weight, based on total weight of the fill liquid composition.

The polyethylene glycol used in the present invention has an average molecular weight of usually 100 to 800, preferably 150 to 700, or more preferably 200 to 600.

The mixing amount of water is usually 0.01% by weight or more and less than 20% by weight, preferably 0.1% by weight or more and less than 20% by weight, or more preferably 1% by weight or more and 18% by weight or less, based on total weight of the fill liquid composition.

The weight ratio of water to polyethylene glycol is usually (0.01 to 20):(99.99 to 80), preferably (0.1 to 20):(99.9 to 80), or more preferably (1 to 20):(99 to 80).

In the present invention, the term "terpenoid " refers to terpenoid and essential oil including terpenoid. Specifically, examples of terpenoid include limonene, pinene, camphene, cymene, cineole, citronellol, geraniol, nerol, linalool, menthol, terpinol, rhodinol, borneol, isoborneol, menthone, camphor, eugenol, cinnzeylanol and so forth, and examples of essential oil including terpenoid include tohi oil, orange oil, peppermint oil, camphor white oil, eucalyptus oil, turpentine oil, lemon oil, ginger oil, clove oil, cinnamon oil, lavender oil, fennel oil, chamomile oil, perilla oil, spearmint oil and so forth. Terpenoid may be used alone or as mixtures of two or more of them. The mixing amount of terpenoid is usually 0.1 to 15% by weight, preferably 0.15 to 10% by weight, more preferably 0.15 to 5% by weight, or especially preferably 0.2 to 2% by weight, based on total weight of the fill liquid compoisition.

Furthermore, in the present invention, the term "menthol" refers to *l*-menthol or *dl*-menthol.

The fill liquid composition of the present invention may use ibuprofen together with other drugs. The drugs other than ibuprofen include antipyretic analgesic, anti-histamine, antitussive, expectorant, sympathetic nerve stimulant, analeptic, hypnotic sedative, antiphlogistic and so forth. These medicinal components may be used alone or as mixtures of two or more of them in combination with ibuprofen.

Hereinafter, some of preferred examples of the medicinal component are presented. However, the present invention should not be limited to the examples.

Preferred antipyretic analgesic includes, for example, various antipyretic analgesics such as aspirin, aspirin aluminum, acetaminophen, ethenezamide, salsalate, salicylamide, lactylphenetidine, and sodium salicylate. The antipyretic analgesic is used in an amount of usually 50 to 1,500% by weight, preferably 75 to 500% by weight, or more preferably 100 to 250% by weight, based on the amount of ibuprofen.

Preferred examples of antihistamine include various antihistamines such as isothipendyl hydrochloride, diphenylpyraline hydrochloride, diphenhydramine hydrochloride, dipheterol hydrochloride, triprolidine hydrochloride, triperenamine hydrochloride, thonzylamine hydrochloride, phenetazine hydrochloride, methozylazine hydrochloride, promethazine hydrochloride, diphenhydramine salicylate, carbubixamine diphenyldisulfonate, alimemazine tartrate, diphenhydramine tannate, diphenylpyraline theoclate, mebhydroline napazisilate, promethazinemethylene disalicylate salt, carbinoxane maleate, chlorophenylamine *dl*-maleate, chlorophenylamine d-maleate, and dipheterol phospate. The antihistamine is used in an amount of usually 0.3 to 1,500% by weight, preferably 0.4 to 500% by weight, or more preferably 0.5 to 250% by weight, based on the amount of ibuprofen.

Preferred antitussive includes, for example, aroclamide hydrochloride, chlopelastin hydrochloride, carbetapentane citrate, tipepidine citrate, sodium dibunate, dextromethorphan hydrobromide, dextromethorphan phenolphthalate salt, tipepidine hibenzate, chloropelastine phendizoate, codeine phosphate, dihydrocodeine phosphate, noscapine hydrochloride, noscapine and so forth. The antitussive is used in an amount of usually 1 to 25% by weight, preferably 3 to 20% by weight, or more preferably 5 to 15% by weight, based on the amount of ibuprofen.

Preferred expectorant includes, for example, potassium guaiacolsulfonate, guaifenesin and so forth. The expectorant is used in an amount of usually 20 to 100% by weight, preferably 30 to 80% by weight, or more preferably 40 to 60% by weight, based on the amount of ibuprofen.

Preferred sympathetic nerve stimulant includes, for example, *dl*-methylephedrine hydrochloride, *dl*-methylephedrine saccharin salt, phenylpropanolamine hydrochloride and so forth. The sympathetic nerve stimulant is used in an amount of usually 5 to 25% by weight, preferably 7.5 to 20% by weight, or more preferably 10 to 15% by weight, based on the amount of ibuprofen.

Preferred analeptic includes, for example, anhydrous caffeine, caffeine, sodium caffeine benzoate and so forth. The analeptic is used in an amount of usually 3 to 75% by weight, preferably 5 to 50% by weight, or more preferably 7 to 25% by weight.

Preferred hypnotic sedative includes, for example, bromovalerylurea and so forth. The hypnotic sedative is used in an amount of usually 5 to 1,500% by weight, preferably 10 to 500% by weight, or more preferably 15 to 250% by weight, based on the amount of ibuprofen.

Preferred antiphlogistic includes, for example, tranexamic acid, glycyrrhizic acid and its analogs. The antiphlogistic is used in an amount of usually 1 to 1,500% by weight, preferably 3 to 500% by weight, or more preferably 5 to 250% by weight, based on the amount of ibuprofen.

The drugs other than ibuprofen may be used in amounts selected from within maximum daily dosage and maximum single dosage prescribed in the case of formulations according to the accepted standards or depending on the administration method and dosage according to non-standard formulations.

The fill liquid composition of the present invention may further comprise those components mixed in oral liquid preparations, such as a solubilizer, a thickening agent, a pH adjuster, and a coloring agent in amounts in which they are mixed generally.

Hereinafter, mention is made of those compounds that are preferably mixed as examples. However, the present invention should not be limited thereto.

The solubilizer may include, for example, propylene glycol, glycerol and so forth. The solubilizer may be used in an amount of usually 1 to 20% by weight, preferably 2 to 15% by weight, or more preferably 4 to 12% by weight, based on the total amount of the fill liquid composition.

The thickening agent includes polyvinylpyrrolidone, hydroxypropylmethylcellulose and so forth. The thickening agent is used in an amount of usually 30% by weight or less, preferably 20% by weight or less, or more preferably 10% by weight or less, based on the total amount of the fill liquid composition.

The pH adjuster includes citric acid, phosphoric acid and their edible salts, sodium hydroxide, potassium hydroxide, sodium hydrogen carbonate and so forth. The pH adjuster is used in an amount of usually 10% by weight or less, preferably 7.5% by weight or less, or more preferably 5% by weight or less, based on the total amount of the fill liquid composition. It is desirable that the pH of the fill liquid composition of the present invention is adjusted 2 to 7, preferably 2 to 6, or more preferably 2 to 5.

The coloring agent includes edible dyes, calomel and so forth. The coloring agent is used in an amount of usually 1% by weight or less, preferably 0.5% by weight or less, or more preferably 0.1% by weight or less, based on the total amount of the fill liquid composition.

The fill liquid composition of the present invention can be obtained by a conventional method. For example, it can be produced by mixing the above-mentioned components in a mixed solution composed of heated water, polyethylene glycol and a solubilizer, followed by heating with stirring. The fill liquid composition of the present invention can be filled in soft capsules or hard capsules and is useful since it can provide means for encapsulating drugs in a solution amount small enough to be easily swallowed down (usually 1 mL or less, preferably 0.7 mL or less, per capsule). After filling it in capsules, the fill liquid composition does not form precipitation of crystals at room temperature or in cold place.

### EXAMPLES

Hereinafter, the present invention will be more concretely explained below with reference to examples.

### Example 1

A mixture of 274 g of polyethylene glycol 400 and 50 g of purified water was heated to about 60°C. To the mixture were added 75 g of ibuprofen and 1 g of *l*-menthol, followed by stirring for dissolution to obtain a colorless transparent solution. The transparent solution was filled in capsules by an ordinary method in an amount of 400 mg per capsule to produce 1,000 hard capsules.

### Examples 2 to 6 and Comparative Examples 1 to 3

In the same method as in Example 1, the components shown in Tables 1 and 2 were used to produce capsules of Examples 2 to 6 and Comparative Examples 1 to 3.

### Example 7

A mixture of 221.4 g of polyethylene glycol 400 and 40 g of purified water was heated to about 60°C. To the mixture were added 75 g of ibuprofen, 0.6 g of d-chlorophenylamine maleate, 10 g of *dl*-methylephedrine hydrochloride, 4 g of dihydrocodeine phosphate, 42 g of guaifenesin, and 7 g of *l*-menthol, followed by stirring for dissolution to obtain a colorless transparent solution. The transparent solution was filled in capsules by an ordinary method in an amount of 400 mg per capsule to produce 1,000 hard capsules.

### Example 8

A mixture of 245 g of polyethylene glycol 400 and 45 g of purified water was heated to about 60°C. To the mixture were added 75 g of ibuprofen, 7 g of anhydrous caffeine, 10 g of *dl*-methylephedrine hydrochloride, 4 g of dextromethorphan hydrobromide, 42 g of guaifenesin, 7 g of *l*-menthol, and 15 g of citric acid, followed by stirring for dissolution to obtain a colorless transparent solution. The transparent solution was filled in capsules by an ordinary method in an amount of 450 mg per capsule to produce 1,000 hard capsules.

### Example 9

A mixture of 228.5 g of polyethylene glycol 400 and 40 g of purified water was heated to about 60°C. To the mixture were added 75 g of ibuprofen, 12.5 g of diphenhydramine hydrochloride, 10 g of *dl*-methylephedrine hydrochloride, 4 g of dihydrocodeine phosphate, 8 g of noscapine hydrochloride, 7 g of *l*-menthol, and 15 g of polyvinylpyrrolidone, followed by stirring for dissolution to obtain a colorless transparent solution. The transparent solution was filled in capsules by an ordinary method in an amount of 400 mg per capsule to produce 1,000 hard capsules.

### Evaluation of Capsules of Examples 1 to 9 and Comparative Examples 1 to 3

The capsules of Examples 1 to 9 and Comparative Examples 1 to 3 were evaluated for their appearance and the influence of the fill liquid on the capsule. The evaluation of appearance was performed by visually observing the appearance of the capsules immediately after production and after storage at -5°C for 2 weeks. The results where no precipitation of crystals of ibuprofen occurred were assigned "O" and the results where precipitation of crystals of ibuprofen occurred were assigned "X". The evaluation of the influence on the capsules was performed by visually observing the state of the capsule after standing the capsule upright at a room temperature for 1 week from the filling of the fill liquid to the capsule. The results where no adverse influence was observed were assigned "O" and the results where a remarkable influence (capsule cracking, or softening of capsule) was observed were assigned "X".

### <Results of Evaluations>

The results obtained are shown in Tables 1 to 3 below.

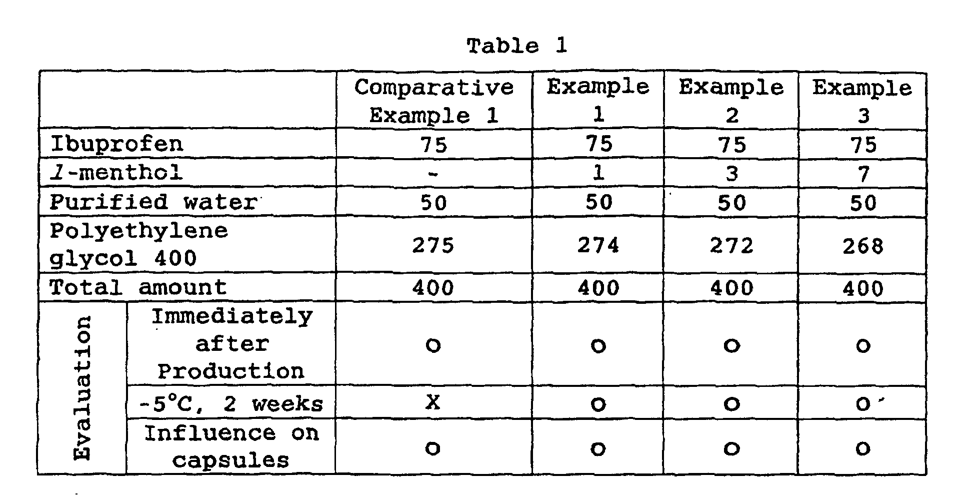

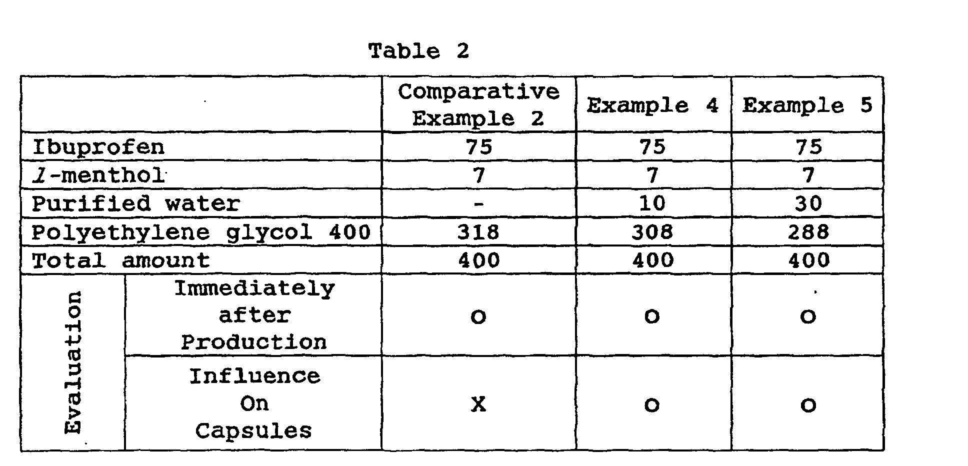

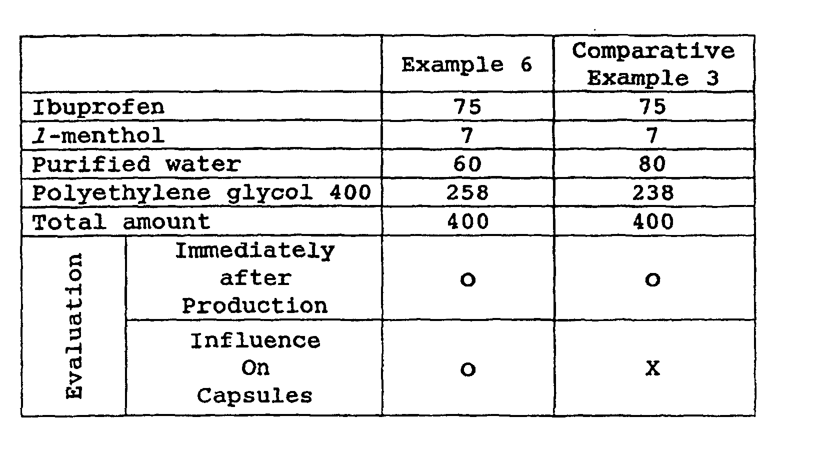

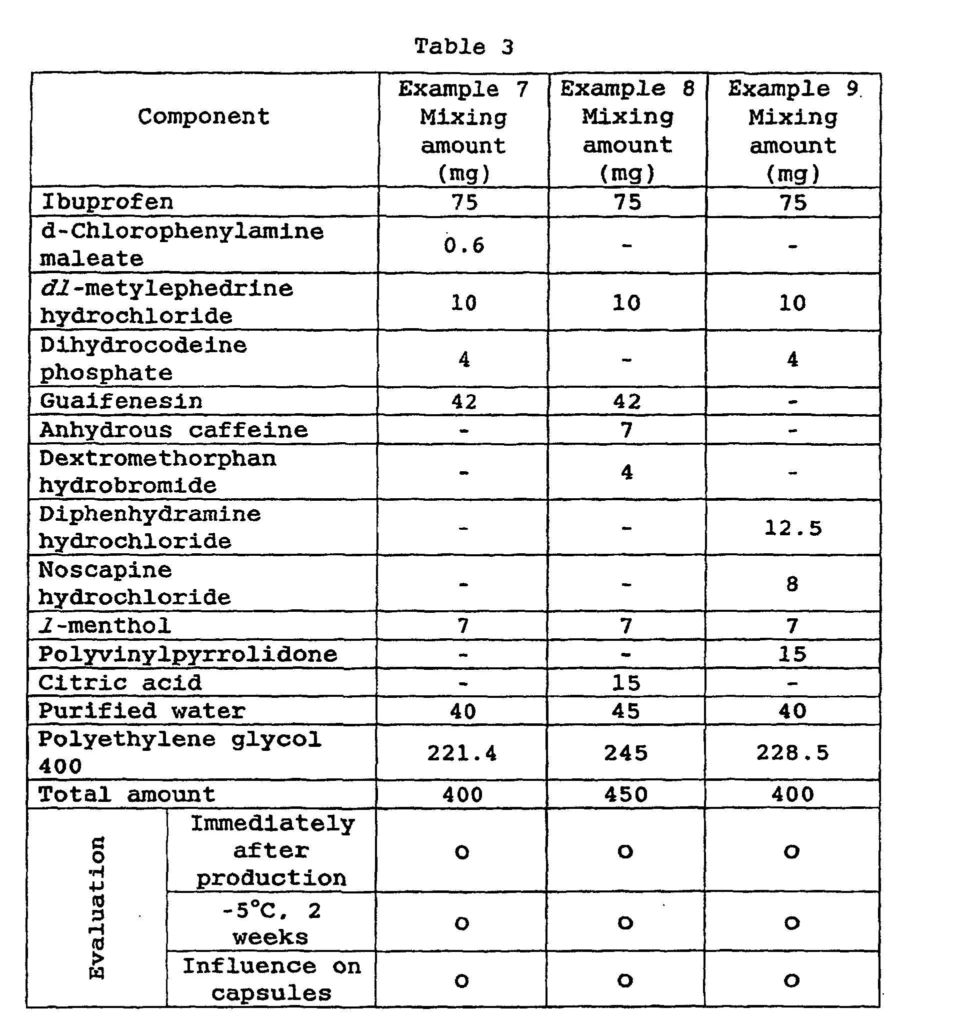

From the comparison between Example 1 to 3 with Comparative Example 1, it can be seen that precipitation of crystals of ibuprofen can be prevented by mixing *l*-menthol in a solution of ibuprofen, polyethylene glycol and water and filling the resulting liquid in a capsule.

Further, from the comparison between Examples 4 to 6 with Comparative Examples 2 and 3, it can be seen that when water is not mixed in the fill liquid composition having dissolved therein ibuprofen, capsule cracking is observed. On the other hand, when the amount of water exceeds 20% by weight based on the amount of the fill liquid composition, softening of the capsule is observed.

As shown in Examples 7 to 9, even when agents such as antipyretic analgesic, anti-histamine, antitussive, expectorant, sympathetic nerve stimulant, central stimulant, hypnotic sedative, and antiphlogistic are mixed in the fill liquid composition having dissolved therein ibuprofen, no precipitation of crystals of ibuprofen was observed.

Except for the capsule of Comparative example 2 that contained no water and the capsule of Comparative Example 3 that contained water in an amount of more than 20% by weight based on the amount of the fill liquid composition, no particular influence of the fill liquid on the capsules was observed.

In the case where the capsule in each Example was produced by using soft capsules instead of hard capsules, similar results were obtained.

### Example 10

Instead of *l*-menthol, Limonene was used to produce 1,000 hard cupsules in the same method as example 1.

### Example 11

Instead of *l*-menthol, borneol was used to produce 1,000 hard cupsules in the same method as example 1.

### Example 12

Instead of *l-*menthol, *dl*-camphor was used to produce 1,000 hard cupsules in the same method as example 1.

### Example 13

Instead of *l*-menthol, peppermint oil was used to produce 1,000 hard cupsules in the same method as example 1.

### <Evaluation of Capsules of Examples 10 to 13>

The capsules of Examples 10 to 13 were evaluated for their appearance after 2 weeks preservation at -5°C. The results obtained are shown in Tables 4 below.

**Table 4**

| | | Example 10 | Example 11 | Example 12 | Example 13 |
|---|---|---|---|---|---|
| Ibupurofen | | 75 | 75 | 75 | 75 |
| Limonene | | 7 | - | - | - |
| Borneol | | - | 7 | - | - |
| *dl*-camphor | | - | - | 10 | - |
| Peppermint oil | | - | - | - | 7 |
| Purified water | | 50 | 50 | 50 | 50 |
| Polyethylene glycol 400 | | 268 | 268 | 265 | 268 |
| Total amount | | 400 | 400 | 400 | 400 |
| | -5°C, 2 weeks | o | o | o | o |

These results suggest that precipitation of crystal of ibuprofen can be prevented by terpenoid or essential oil including terpenoid as well as by *l*-menthol.

### INDUSTRIAL APPLICABILITY

A fill liquid composition and a capsule preparation that form no precipitation of crystals of ibuprofen when water is mixed therein can be provided by the present invention. The capsule of the present invention exhibits effects that the prevention of bitterness of ibuprofen and the quick-activeness of ibuprofen can be consistently achieved.

## Claims

1. A fill liquid composition for capsules comprising ibuprofen, polyethylene glycol, water and terpenoid.

2. The fill liquid composition according to claim 1, wherein the content of terpenoid is 0.1 to 15% by weight based on total weight of the fill liquid composition.

3. The fill liquid composition according to claim 1 or 2, wherein the terpenoid is selected from the group consisting of menthol, limonene, borneol, *dl*-camphor, and peppermint oil.

4. The fill liquid composition according to claim 3, wherein terpenoid is menthol or peppermint oil.

5. The fill liquid composition according to any one of claims 1 to 4, further comprise one or more drugs selected from the group consisting of antipyretic analgesic, anti-histamine, antitussive, expectorant, sympathetic nerve stimulant, central stimulant, hypnotic sedative, and antiphlogistic.

6. An ibuprofen capsule preparation comprising a capsule and the fill liquid composition according to any one of claims 1 to 5 filled in the capsule.

## Patentansprüche

1. Zusammensetzung einer Füllflüssigkeit für Kapseln, enthaltend Ibuprofen, Polyethylenglykol, Wasser und ein Terpenoid.

2. Zusammensetzung einer Füllflüssigkeit nach Anspruch 1, wobei der Terpenoidgehalt 0,1 bis 15 Gewichtsprozent auf der Basis des Gesamtgewichts der Zusammensetzung der Füllflüssigkeit ist.

3. Zusammensetzung einer Füllflüssigkeit nach Anspruch 1 oder 2, wobei das Terpenoid aus der Gruppe ausgewählt ist, die aus einem Menthol, Limonen, Borneol, *dl*-Kampfer und einem Pfefferminzöl besteht.

4. Zusammensetzung einer Füllflüssigkeit nach Anspruch 3, wobei das Terpenoid ein Menthol oder ein Pfefferminzöl ist.

5. Zusammensetzung einer Füllflüssigkeit nach einem der Ansprüche 1 bis 4, welche des Weiteren ein oder mehrere Arzneimittel enthält, die aus der Gruppe ausgewählt sind, die aus einem antipyretischen Analgetikum, einem Anti-Histamin, einem Hustenmittel, einem schleimlösenden Mittel, einem Stimulanz der sympathischen Nerven, einem zentralen Stimulanz, einem hypnotischen Sedativum und einem Antiphlogistikum besteht.

6. Ibuprofenkapselpräparat mit einer Kapsel und der in die Kapsel gefüllten Zusammensetzung einer Füllflüssigkeit nach einem der Ansprüche 1 bis 5.

## Revendications

1. Composition liquide de remplissage pour gélules contenant de l'ibuprofène, du polyéthylène glycol, de l'eau et un composé terpénique.

2. Composition liquide de remplissage selon la revendication 1,
**caractérisée en ce que**
le contenu en composé terpénique est de 0,1 à 15 % en poids par rapport au poids total de la composition liquide de remplissage.

3. Composition liquide de remplissage selon la revendication 1 ou 2,
**caractérisée en ce que**
le composé terpénique est choisi dans le groupe comprenant le menthol, le limonène, le bornéol, le *dl*-camphre et l'huile de menthe poivrée.

4. Composition liquide de remplissage selon la revendication 3,
**caractérisée en ce que**
le composé terpénique est le menthol et l'huile de menthe poivrée.

5. Composition liquide de remplissage selon l'une quelconque des revendications 1 à 4,
**caractérisée en ce qu'**
elle comprend en plus un ou plusieurs médicaments choisis dans le groupe comprenant un analgésique antipyrétique, un anti-histamine, un antitussif, un expectorant, un stimulant du nerf sympathique, un stimulant central, un analeptique, un sédatif hypnotique et un antiphlogistique.

6. Préparation d'une gélule d'ibuprofène comprenant une gélule et la composition liquide de remplissage selon l'une quelconque des revendications 1 à 5 qui remplit la gélule.
